# EUROPÄISCHE PATENTSCHRIFT

(11) **EP 1 265 592 B1**
(45) Veröffentlichungstag und Bekanntmachung des Hinweises auf die Patenterteilung: **21.02.2007**
(21) Anmeldenummer: 01923512.6
(22) Anmeldetag: 08.03.2001
(51) Int. Cl.: A61K 8/97, A61Q 1/12, A61Q 17/04, A61Q 19/04

(54) **KOSMETISCHER KOMPAKT- ODER CREMEPUDER**
COSMETIC COMPACT OR CREME POWDER
POUDRE-CREME OU POUDRE COMPACTE UTILISEE EN COSMETIQUE

(30) Priorität: 23.03.2000 DE 10015363
(43) Veröffentlichungstag der Anmeldung: 18.12.2002
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2001/000953
(87) Internationale Veröffentlichungsnummer: WO 2001/070186

(56) Entgegenhaltungen:
- FR-A- 2 787 711
- US-A- 5 559 146
- "Faking it without DHA" SOAP, PERFUMERY & COSMETICS, Bd. 69, Nr. 9, September 1996 (1996-09), Seiten 33-34, XP002174759
- DATABASE WPI Week 199405 Derwent Publications Ltd., London, GB; AN 1994-040024 XP002174760 & JP 05 345718 A (NIPPON SHINYAKU & SUNSTAR CHEM), 27. Dezember 1993 (1993-12-27)

## Beschreibung

Die Erfindung betrifft einen kosmetischer Kompakt- oder Cremepuder, der selbstbräunende und gegebenenfalls lichtschützende Eigenschaften aufweist.

Es ist bekannt, Selbstbräunungsmittel auf Basis von Dihydroxyaceton (DHA) in Form von verschiedenen flüssigen Emulsionen oder Gelen herzustellen. In vielen Fällen bedarf es jedoch der Stabilisierung durch andere Zusatzstoffe. So ist in der EP-B-752842 eine DHA-Zusammensetzung mit bestimmten Sulfitsalzen sowie gegebenenfalls Aminosäuren beschrieben. In der WO96/3119 werden Kombinationen von DHA mit Lecithinen und gegebenenfalls Sterolen zwecks Stabilisierung in wäßriger Lösung erwärmt und anschließend liposomal verkapselt.

Aus Soap, Perfumery & Cosmetics, Bd. 69, Nr.9 (1996) S. 33-34 ist eine Selbstbräunungsmilch bekannt, die neben Mahakanni STLC als Bräunungskomponente auch den Einsatz von DHA und bestimmten W-Absorbern wie Thulasi Ashvini root oil und pTpT beschreibt.

Bei der Herstellung von Preßpudern oder Cremepudern besteht das Problem, daß diese normalerweise Wachsanteile enthalten, die auf 60 bis 85 °C erhitzt werden müssen, um die anderen Formulierungsbestandteile einbringen zu können. Bei diesen hohen Temperaturen finden verstärkt Teilreaktionen des DHA mit anderen Komponenten statt, wodurch unterschiedliche Verluste der Bräunungsfähigkeit auftreten. Bisher wurde von der Herstellung derartiger Puder weitgehend abgesehen, und es gibt keine entsprechenden Produkte auf dem Markt.

Die Aufgabe der Erfindung besteht darin, Kompakt- oder Cremepuder mit sehr guter Farbgebung auf Basis von Selbstbräunungsmitteln bereitzustellen, die in stabiler homogener Verteilung vorliegen und dabei den Anteil des eingebrachten Dihydroxyacetons im wesentlichen voll für die Farbgebung zu nutzen.

Erfindungsgemäß besteht der kosmetische Kompakt- oder Cremepuder aus einem Selbstbräunungsmittel, umfasend ein Gemisch von Dihydroxyaceton (DHA) mit Mahakanni(Eclipta alba Hassk)-Extrakt und Tulpenextrakt, weiteren farbgebenden Pigmenten, einem Wachs oder Gemisch mehrerer Wachse, einer Pudergrundlage und gegebenenfalls weiteren kosmetischen Hilfs- und Wirkstoffen in stabiler homogener Verteilung, wobei der Mahakanni-Extrakt ein wäßriger Extrakt ist, enthaltend 2-Hydroxy-1,4-naphthochinon und Eumelanin, und der Tulpenextrakt ein wäßriger Extrakt aus UV-bestrahlten Tulpen ist, enthaltend Dimere von Thymin und Pyrimidinen und Gemische davon.

Mahakanni ist ein aus der Mahakanni-Pflanze (Eclipta alba Hassk) gewonnener natürlicher Bräunungsfarbstoff, der im wesentlichen aus 2-Hydroxy-1,4-naphthochinon und Eumelanin besteht und der zu einem besonders naturnahen Braunton der Haut führt. Die genaue lateinische Bezeichnung lautet Eclipta alba Hassk(Syn)Eclipta prostata.

Noch verstärkt werden kann der Selbstbräunungseffekt durch Zugabe eines Tulpenextraktes (Tulipa ; von Garten- und Wildtulpen, insbesondere von DNA-reichen Tulpen-Arten). Dabei handelt es sich um einen Extrakt mit hohen Anteilen an Dimeren von Nucleotiden, der unter der Bezeichnung TULIPA NUCLEOTIDS von Greentech, St. Beauzire, Frankreich, hergestellt und vertrieben wird. Der Extrakt enthält hohe Anteile im Bereich von 8 bis 40 Gew-% von Dimeren von Thymin und/oder Pyrimidinen und zeigt einen deutlichen Pigmentierungseffekt auf der Haut.

Der Gehalt an 2-Hydroxy-1,4-naphthochinon liegt vorteilhaft im Bereich von 37-42 Gew-% und der Gehalt von Eumelanin im Bereich von 47-52 Gew-%, bezogen auf den Gesamtgehalt der in dem Mahakanni-Extrakt vorhandenen Komponenten.

Der Mahakanni-Extrakt enthält weiterhin Ascorbinsäure, Lecithine, Sphingomyelin, β-Caroten und Phosphosphingolipid zusammen im Konzentrationsbereich von 7-10 Gew-%, bezogen auf das Gesamtgewicht des Extraktes.

Der Mahakanni-Extrakt, der vorzugsweise in Verkapselung durch Liposomen vorliegt, kann in Konzentrationen von 0,5 bis 10 **Gew-%** eingesetzt werden, bezogen auf das Gesamtgewicht des Puders. Der Extrakt hat einen Brechungsindex von 1,4-1,5 und enthält keine Isoflavonoide.

Der Tulpenextrakt, der auch verkapselt in Liposomen vorliegen kann, wird in Konzentrationen von 0,5 bis 10 Gew-% in den Puder eingebracht, bezogen auf das Gesamtgewicht des Puders. Die Konzentration.

Die Konzentration von DHA liegt im Bereich von 0,1 bis 5 Gew-%, bezogen auf das Gesamtgewicht des Puders.

Der erfindungsgemäße Puder kann weiterhin ein Sonnenschutzmittel in Form eines UV-Filters enthalten.

Mit Hilfe der Erfindung ist es möglich, im wesentlichen den gesamten Anteil des Selbstbräunungsmittels formulierungswirksam zu machen und Verluste bzw. Nebenreaktionen des DHA zu vermeiden oder sehr stark zu verzögern. Insbesondere die Einhaltung des pH-Wertes der Vormischung: Pigment-Teilgemisch, DHA und gegebenenfalls Sonnenschutzmittel trägt wesentlich zur problemlosen Einarbeitung des Gemisches und zum Erhalt einer stabilen Formulierung bei. Der pH-Wert kann durch Zusatz bekannter und kosmetisch annehmbarer pH-Regulatoren, wie EDTA, Essigsäure, Citronensäure usw. entsprechend beeinflußt werden.

Weiterhin wird die Genauigkeit des Anteils der farbgebenden Komponenten, insbesondere von DHA, durch Vermeidung von Reaktionsverlusten wesentlich erhöht, wenn nicht überhaupt erst für eine nahezu exakt einstellbare Konzentration ermöglicht.

Der Gehalt an Selbstbräunungsmitteln liegt insgesamt im Bereich von 1 bis 15 Gew-%, bezogen auf die Gesamtzusammensetzung. Dabei liegen höchstens jeweils 5 Gew-% DHA, 10 Gew-% Mahakanni-Extrakt und 10 Gew-% Tulpenextrakt im Gemisch vor. Die Extrakte von Mahakanni und Tulpe sind untereinander beliebig mischbar und werden je nach gewünschter Brauntönung festgelegt. Das gleiche betrifft die Mischbarkeit der beiden Extrakte mit DHA.

Als Pigmente, Pigmentgemische oder Pulver mit pigmentartiger Wirkung, worunter auch solche mit Perlglanz-Effekt zu verstehen sind, können zum Beispiel ausgewählt werden Eisenoxide, natürliche Aluminiumsilicate wie Ocker, Titandioxid, Zinkoxid, Siliciumdioxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff.

Für die wäßrig-alkoholische Lösung oder Suspension der Pigmente werden einwertige Alkohole verwendet, beispielsweise Ethanol, Isopropanol, n-Propanol oder Gemische davon. Es können auch mehrwertige Alkohol eingesetzt werden, beispielsweise Glycerin, ein Propandiol oder ein Butandiol, vorteilhaft auch ein Gemisch von zwei oder mehreren.

Die Alkoholkonzentration liegt dabei im Bereich von 1 bis 10 Vol%, bezogen auf die Lösung oder Suspension der Pigmente.

Als organisches Sonnenschutzmittel (Filter) wird vorzugsweise Octyl Methoxycinnamate eingesetzt. Andere geeignete Filter sind Benzophenone-3, Butyl Metoxybenzoylmethan oder 4-Methylbenzylidene Camphor oder Gemische davon. Die Konzentration dieser Filter kann im Bereich von 1 bis 10 Gew-% liegen, bezogen auf das Gesamtgewicht des Puders. Es wurde gefunden, daß bei gleichzeitigem Vorhandensein von DHA und einem Sonnenschutzmittel der genannten Typen eine sehr gute Stabilisierung des DHA erfolgt.

Zusätzlich zu dem alkohollöslichen organischen Sonnenschutzfilter kann ein wasserlöslicher organischer Sonnenschutzfilter enthalten sein. Dazu gehören beispielsweise Benzophenone-3 und Phenylbenzimidazole Sulfonic Acid. Die Konzentration dieser wasserlöslichen Filter kann im Bereich von 2 bis 15 Gew-% liegen.

Der erfindungsgemäße Puder kann mit den entsprechenden Lichtschutzfiltern einen Lichtschutzfaktor von 5 bis 15 haben.

Der erfindungsgemäße Puder kann weitere kosmetische Wirkstoffe enthalten, wie beispielsweise Antioxidationsmittel oder Radikalfänger, Konservierungsmittel, feuchthaltende Substanzen, Duftstoffe, polare und unpolare öle, Polymere, Copolymere, Emulgatoren, Stabilisatoren, pflanzliche Wirkstoffe, Polymere, entzündungswidrige natürliche Wirkstoffe.

Zu Antioxidationsmitteln gehören Vitamine wie Vitamin C und Derivate davon, beispielsweise Ascorbylacetat, -phosphat und - palmitat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate usw.

Ein besonders vorteilhaftes Gemisch aus Enzymen und Vitaminen ist ein durch Ultraschallbehandlung hergestelltes Aufschlußprodukt einer Hefe, wobei das Aufschlußprodukt Superoxiddismutase (SOD), Protease, Vitamin B₂, Vitamin B₆, Vitamin B₁₂, Vitamin D₂ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Besonders vorteilhaft für die Herstellung des Enzym-/Vitamingemisches ist ein Aufschlußverfahren mittels Ultraschall, das in der DE 4241154C1 beschrieben ist und bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht. Dabei hat die Sonotrode einen Winkel von 80,5 bis 88,5°, bezogen auf die Eintrittsebene der Sonotrode, und das

Verhältnis der Eintauchlänge der Sonotrode in mm zum Beschallungsvolumen in ml wird auf einen Wert von 1:1, 1 bis 1:20 eingestellt. Der Feststoffanteil in dem zu beschallenden Medium liegt im Bereich von 1:0,02 bis 1:2,2 (in Gew.-%).

Als Zelldispersion können Hefen, wie Bäckerhefe, Brauereihefe, Weinhefe sowie besonders behandelte Hefen, wie z.B. SOD-angereicherte Hefen, eingesetzt werden. Eine vorteilhaft einzusetzende Zelldispersion enthält z. B. Saccharomyces cerevisiae.

Als weiteren Wirkstoff kann das Präparat vorteilhaft Kaolin gemäß WO96/17588 enthalten, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der modifizierte Kaolin kann einen Anteil von 0,1 bis 6 Gew-% haben, bezogen auf die Gesamtmenge des Präparates.

Die für die Erfindung eingesetzten öle können übliche kosmetische öle sein, wie ein Mineralöl; hydriertes Polyisobuten; synthetisches oder aus Naturprodukten hergestelltes Squalan (INCI-Name: Squalane, z.B. Synthesqual^{®}, Cosbiol^{®}); kosmetische Ester oder Ether, die verzweigt oder unverzweigt, gesättigt oder ungesättigt sein können; pflanzliche öle; oder Gemische zweier oder mehrerer davon.

Je nachdem welche öle ausgewählt werden, werden die kosmetischen Eigenschaften der festen Zusammensetzung beeinflußt, wie Transparenzgrad, Weichheit, Härte, Spreitungswirkung. Bevorzugt sind dickflüssige öle.

Als Wachse in dem erfindungsgemäßen Puder können eingesetzt werden natürliche pflanzliche Wachse, tierische Wachse, natürliche und synthetische Mineralwachse und synthetische Wachse. Dazu gehören beispielsweise Carnaubawachs, Candellilawachs, Bienenwachs, Wollwachs, Hartparaffin, Ceresin, Ozokerit, Silicone, Polyethylenglycol- oder -glycolesterwachse.

Pudergrundlagen sind Festteilchen mit nahezu abgerundetem Korn und Teilchengrößen von etwa 25-110 µm und umfassen beispielsweise Silicate wie Kaolin, Aerosil, Talkum; Carbonate wie Magnesiumcarbonat, Calciumcarbonat; Oxide wie Zinkoxid, Titanoxid; Stearate wie Zinkstearat, Magnesiumstearat, Aluminiumstearat; Stärke; Eiweißabbauprodukte wie gepulverte Seide; Labilin; synthetische organische Polymerisationsprodukte; Zink- oder Magnesiumdecanat.

Der Wassergehalt der erfindungsgemäßen Cremes beträgt nicht mehr als 15 Gew-%, vorzugsweise nicht mehr als 12 Gew-%.

Die Erfindung betrifft auch ein Verfahren zur Herstellung des Kompakt- oder Cremepuders durch Vermischen der Pudergrundlage und gegebenenfalls weiterer kosmetischer Hilfs- und Wirkstoffe und einem ersten Anteil von 90 bis 50 Gew-% der farbgebenden Pigmente mit dem geschmolzenen Wachs oder Wachsgemisch, anschließender Zugabe des Selbstbräunungsmittels, ausgewählt unter Gemischen von Dihydroxyaceton (DHA) mit Mahakanni-Extrakt und Gemischen von DHA mit Mahakanni-Extrakt und Tulpenextrakt, und einem zweiten Anteil von 10 bis 50 Gew-% der Pigmente in einer wäßrig-alkoholischen Suspension mit einem pH-Wert im Bereich von 3-4 in das geschmolzene Wachsgemisch bei einer Temperatur von 65 bis 70°C, Rühren des Gesamtgemisches bis zur Homogenität und Abkühlen des Gemisches unter Erhaltung der Homogenität.

Die Einhaltung des pH-Wertes bei der Zugabe der Suspension ist besonders kritisch.

Die Abkühlung des Gemisches erfolgt vorteilhaft unter mäßigem Rühren und gleichmäßig mit einer Abkühlgeschwindigkeit von 1-2 °C/Min, um eine Kristallisation des Wachses zu vermeiden und eine homogene Mischung beizubehalten.

Die Erfindung soll nachstehend durch Beispiele näher erläutert werden. Alle Angaben erfolgen in Gewichtsprozent, sofern nichts anderes angegeben ist.

### Beispiel 1 Selbstbräunender Cremepuder I

| | |
|---|---|
| Ceraflex 3547 | 1,5 |
| Ozokerite Wax SP 1020 | 2 |
| Candelilla Wax Substitute | 3 |
| synchrowax HGLC | 2,3 |
| Acide B glycerrhetinique | 0,01 |
| Nipasol M | 0,2 |
| Nipagine M | 0,4 |
| α-Tocopherolacetat | 0,5 |
| Cosmacol EOI | 10 |
| TiO₂ | 5 |
| Eisenoxid Red 7054 | 0,4 |
| ZnO | 4 |
| Cogilor Ochre 90076 | 1,5 |
| Black 1557 | 0,14 |
| SiO₂ | 4 |
| Crodamol Osu | 6 |
| modifiziertes* Kaolin | 2,5 |
| Orgasol 20002Extd Nat Cos | 2 |
| Boron Nitride CCS102 | 2 |
| Dryflo Plus | 10 |
| Talcum | q.s. ad 100 |
| Siliconöl | 6 |
| Mahakanni Extract STLC⁺ | 2,5 |
| DHA | 2,5 |
| Octyl Methoxycinnamate | 1,0 |
| Parfüm | 0,5 |
| Wasser | 12 |
| Biophilic H | 3 |
| Citronensäure | q.s. |

| | |
|---|---|
| * gemäß WO96/17588 ⁺ Campo Research & Development Systems, Singapore | |

### Arbeitsweise :

Ein Gemisch der Pigmente TiO₂, ZnO, Eisenoxide, SiO₂, Black und Ocker wurde in fester Form durch leichtes Vermischen hergestellt und in zwei Chargen von 65 % (Pigmentcharge 1) und 35 % (Pigmentcharge 2) geteilt.

Die Pigmentcharge 2 wurde in einer 3,5 Vol-%igen wäßrig-alkoholischen Lösung, die DHA und den Mahakanni-Extrakt enthielt, zusammen mit dem Sonnenschutzfilter (hier: Octyl Methoxycinnamate) und dem Antioxidationsmittel sowie dem Parfüm zu einer Suspension bei einer Temperatur von etwa 30°C verrührt. Dabei wurde der pH-Wert mit Citronensäure auf etwa 3,5 eingestellt.

Das Wachsgemisch wurde auf eine Schmelztemperatur von 75 bis 80 °C erhitzt und nacheinander darin die Pigmentcharge 2, die öle, Kaolin, Talkum, Bornitrid und die Konservierungsmittel verrührt. Nach Erhalt einer weitgehend homogenen Masse wurde bei etwa 67-69 °C die obige Suspension unter Rühren langsam hinzugegeben.

Schließlich wurde das Gemisch bei 10000 - 15000 U/min bis zur Homogenität gerührt und dann auf Umgebungstemperatur abgekühlt mit einer Abkühlgeschwindigkeit von 1,5 °C/Min und unter mäßigem Rühren. Man erhielt eine in Formen preßbare Pudermasse mit ausgezeichneter Farbstabilität hinsichtlich des Langzeitbräunungseffektes durch DHA und Mahakanni, wobei ein sehr natürlicher Braunton erzielt wurde.

### Beispiel 2 Selbstbräunender Cremepuder II

| | |
|---|---|
| Ceraflex 3547 | 2 |
| Ozokerite Wax SP 1020 | 2 |
| Candelilla Wax Substitute | 2,5 |
| Synchrowax HGLC | 2,3 |
| Nipasol M | 0,2 |
| Nipagine M | 0,4 |
| α-Tocopherolacetat | 0,5 |
| Cosmacol EOI | 8 |
| Isopropyl Palmitate | 8 |
| TiO₂ | 5 |
| Eisenoxid Red 7054 | 0,4 |
| ZnO | 4 |
| Cogilor Ochre 90076 | 1,5 |
| Black 1557 | 0,14 |
| SiO₂ | 4 |
| Crodamol Osu | 7 |
| modifiziertes* Kaolin | 2,5 |
| Orgasol 20002Extd Nat Cos | 2 |
| Boron Nitride CCS102 | 2 |
| Dryflo Plus | 10 |
| Talcum | q.s. ad 100 |
| Siliconöl | 6 |
| DHA | 2,5 |
| Mahakanni Extract STLC | 3,5 |
| Tulpenextrakt⁺ | 2,5 |
| Octyl Methoxycinnamate | 3 |
| Benzophenone-3 | 5,5 |
| Parfüm | 0,5 |
| Wasser | 12 |
| Biophilic H | 3 |

| | |
|---|---|
| ⁺ Tulipa nucleotids, Greentech, St. Beauzire, Frankreich. * gemäß WO96/17588 | |

Es wurde ähnlich wie im Beispiel 1 gearbeitet. Die Pigmentcharge 2 hatte einen Anteil von 28 Gew-%. Der pH-Wert der wäßrig-alkoholischen Suspension betrug 3,8. Man erhielt eine preßfähige Pudermasse mit sehr guter Farbstabilität und Farbtiefe trotz verringerter Anteile Selbstbräunungsmittel.

### Beispiel 3 Kompaktpuder

| | |
|---|---|
| Ceraflex 3547 | 1,5 |
| Ozokerite Wax SP 1020 | 0,8 |
| Candelilla Wax Substitute | 0,6 |
| Nipasol M | 0,2 |
| Nipagine M | 0,4 |
| α-Tocopherolacetat | 0,5 |
| Cosmacol EOI | 1 |
| TiO₂ | 5 |
| Eisenoxid Red 7054 | 0,4 |
| ZnO | 0,2 |
| Cogilor Ochre 90076 | 1,5 |
| Black 1557 | 0,14 |
| modifiziertes* Kaolin | 4 |
| Talcum | q.s. ad 100 |
| Mahakanni Extract STLC | 2,5 |
| Tulpenextrakt | 2,5 |
| DHA | 2,5 |
| Parfüm | 0 , 5 |
| Wasser | 12 |
| Biophilic H | 3 |

| | |
|---|---|
| * gemäß WO96/17588 | |

Es wurde wie im Beispiel 1 gearbeitet. Die Pigmentcharge 2 hatte einen Anteil von 40 Gew-%. Der pH-Wert der wäßrig-alkoholischen Suspension betrug 3,3.

## Patentansprüche

1. Kosmetischer Kompakt- oder Cremepuder, **gekennzeichnet durch** ein Selbstbräunungsmittel, umfassend ein Gemisch von Dihydroxyaceton, Mahakanni-Extrakt(Eclipta alba Hassk) und Tulpenextrakt, weiteren farbgebenden Pigmenten, einem Wachs oder Gemisch mehrerer Wachse, einer Pudergrundlage und gegebenenfalls weiteren kosmetischen Hilfs- und Wirkstoffen, in stabiler homogener Verteilung,
wobei der Mahakanni-Extrakt ein wäßriger Extrakt ist, enthaltend 2-Hydroxy-1,4-naphthochinon und Eumelanin, und der Tulpenextrakt ein wäßriger Extrakt aus UV-bestrahlten Tulpen ist, enthaltend Dimere von Thymin und Pyrimidinen und Gemische davon.

2. Kosmetischer Kompakt- oder Cremepuder nach Anspruch 1, **dadurch gekennzeichnet, daß** der Gehalt an 2-Hydroxy-1,4-naphthochinon im Bereich von 37-42 Gew-% liegt und der Gehalt von Eumelanin im Bereich von 47-52 Gew-% liegt, bezogen auf den Gesamtgehalt der in dem Extrakt vorhandenen Komponenten.

3. Kosmetischer Kompakt- oder Cremepuder nach Anspruch 1, **dadurch gekennzeichnet, daß** das Selbstbräunungsmittel im Gemisch mit einem Sonnenschutzmittel vorliegt.

4. Kosmetischer Kompakt- oder Cremepuder nach Anspruch 1, **dadurch gekennzeichnet, daß** das Sonnenschutzmittel ein organisches alkohollösliches Sonnenschutzmittel ist.

5. Kosmetischer Kompakt- oder Cremepuder nach Anspruch 3 oder 4, **dadurch gekennzeichnet, daß** das Sonnenschutzmittel in einem Anteil von 1 bis 10 Gew-% vorliegt, bezogen auf das Gesamtgemisch des Puders.

6. Kosmetischer Kompakt- oder Cremepuder nach einem der Ansprüche 3 bis 5, **dadurch gekennzeichnet, daß** das Gemisch von Sonnenschutzmittel, Selbstbräunungsmittel und Pigmente einen Pigmentanteil von 25-40 Gew-% enthält, bezogen auf das Gesamtgemisch des Puders.

7. Kosmetischer Kompakt- oder Cremepuder nach einem der Ansprüche 1 bis 6, **dadurch gekennzeichnet, daß** der Wassergehalt des Puders nicht mehr als 15 Gew-% beträgt.

8. Kosmetischer Kompakt- oder Cremepuder nach einem der Ansprüche 1 bis 7, **dadurch gekennzeichnet, daß** der Gehalt an Selbstbräunungsmitteln insgesamt im Bereich von 1 bis 15 Gew-% liegt, bezogen auf die Gesamtzusammensetzung.

9. Kosmetischer Kompakt- oder Cremepuder nach Anspruch 7, **dadurch gekennzeichnet, daß** der maximale Gehalt an DHA bei 5 Gew-%, der von Mahakanni-Extrakt bei 10 Gew-% und der von Tulpenextrakt bei 10 Gew-% im Gesamtgemisch beträgt.

10. Verfahren zur Herstellung des Kompakt- oder Cremepuders nach Anspruch 1, **gekennzeichnet durch**
Vermischen der Pudergrundlage und gegebenenfalls weiterer kosmetischer Hilfs- und Wirkstoffe und einem ersten Anteil von 90 bis 50 Gew-% der farbgebenden Pigmente mit dem geschmolzenen Wachs oder Wachsgemisch, anschließender Zugabe des Selbstbräunungsmittels, ausgewählt unter Gemischen von Dihydroxyaceton (DHA) mit Mahakanni (Eclipta alba Hassk)-Extrakt und Gemischen von DHA mit Mahakanni-Extrakt und Tulpenextrakt, und einem zweiten Anteil von 10 bis 50 Gew-% der Pigmente in einer wäßrig-alkoholischen Suspension mit einem pH-Wert im Bereich von 3-4 in das geschmolzene Wachsgemisch bei einer Temperatur von 65 bis 70 °C, Rühren des Gesamtgemisches bis zur Homogenität und Abkühlen des Gemisches unter Erhaltung der Homogenität.

## Claims

1. A cosmetic compact or creme powder which comprises a self-tanning agent selected from among mixtures of dihydroxy acetone, mahakanni (Eclipta alba Hassk) extract and tulips extract, additional chromophore pigments, a wax or a mixture of several waxes, a powder base and optionally additional cosmetic auxiliaries and active agents in stable homogeneous distribution, the mahakanni extract being an aqueous extract containing 2-hydroxy-1,4-naphthochinon and eumelanin and the tulips extract being an aqueous extract from tulips exposed to UV radiation containing thymine and pyrimidine dimers and mixtures thereof.

2. A cosmetic compact or creme powder according to claim 1 wherein 2-hydroxy-1,4-naphthochinon is contained in the range of 37-42 % by weight and eumelanin is contained in the range of 47-52 % by weight relative to the total content of the constituents contained in the extract.

3. A cosmetic compact or creme powder according to claim 1 wherein the self-tanning agent is mixed with a sunscreen.

4. A cosmetic compact or creme powder according to claim 1 wherein the sunscreen is an organic alcohol-soluble sunscreen.

5. A cosmetic compact or creme powder according to claim 3 or 4 wherein the sunscreen is contained in the range of 1-10 % by weight relative to the entire powder mixture.

6. A cosmetic compact or creme powder according to one of claims 3 to 5 wherein the mixture made up of sunscreen, self-tanning agent and pigments contains pigments in the range of 25-40 % by weight relative to the entire powder mixture.

7. A cosmetic compact or creme powder according to one of claims 1 to 6 wherein the water content of the powder does not exceed 15 % by weight.

8. A cosmetic compact or creme powder according to one of claims 1 to 7 wherein the total content of self-tanning agents is in the range of 1-15 % by weight relative to the entire composition.

9. A cosmetic compact or creme powder according to claim 7 wherein the maximum content of DHA is 5 % by weight, the maximum content of mahakanni extract is 10 % by weight and the maximum content of tulips extract is 10 % by weight relative to the total mixture.

10. A method for manufacturing the compact or creme powder according to claim 1 which comprises the steps of mixing the powder base and optionally additional cosmetic auxiliaries and active agents and a first portion of 90-50 % by weight of the chromophore pigments with the molten wax or wax mixture, subsequently adding the self-tanning agent selected from among mixtures of dihydroxy acetone (DHA) and mahakanni (Eclipta alba Hassk) extract and mixtures of DHA, mahakanni extract and tulips extract and a second portion of 10-50 % by weight of the pigments in an aqueous-alcoholic suspension having a pH value ranging from 3 to 4 into the molten wax mixture at a temperature of 65-70 °C, stirring the entire mixture until it is homogeneous and cooling the mixture while maintaining its homogeneity.

## Revendications

1. Poudre-crème ou poudre compacte utilisée en cosmétique, **caractérisée par** un agent autobronzant comprenant un mélange de dihydroxyacétone, d'extrait de mahakanni (Eclipta alba Hassk.) et d'extrait de tulipe, d'autres pigments colorants, d'une cire ou d'un mélange de plusieurs cires, d'une base de poudre et le cas échéant d'autres adjuvants et substances actives utilisées en cosmétique, en dispersion homogène,
sachant que l'extrait de mahakanni est un extrait aqueux contenant de la 2-hydroxy-1,4-naphtoquinone et de l'eumélanine et que l'extrait de tulipe est un extrait aqueux de tulipes soumises à un rayonnement ultraviolet contenant des dimères de thymine et de pyrimidines et des mélanges de ceux-ci.

2. Poudre-crème ou poudre compacte utilisée en cosmétique selon la revendication 1, **caractérisée en ce que** la teneur en 2-hydroxy-1,4-naphtoquinone se situe dans la plage de 37 à 42 % en poids et que la teneur en eumélanine se situe dans la plage de 47 à 52 % en poids, par rapport à la teneur totale des composés présents dans l'extrait.

3. Poudre-crème ou poudre compacte utilisée en cosmétique selon la revendication 1, **caractérisée en ce que** l'agent autobronzant est présent en mélange avec un écran solaire.

4. Poudre-crème ou poudre compacte utilisée en cosmétique selon la revendication 1, **caractérisée en ce que** l'écran solaire est un écran solaire organique soluble dans l'alcool.

5. Poudre-crème ou poudre compacte utilisée en cosmétique selon la revendication 3 ou 4, **caractérisée en ce que** l'écran solaire est présent dans une proportion de 1 à 10 % en poids par rapport au mélange total de poudre.

6. Poudre-crème ou poudre compacte utilisée en cosmétique selon l'une quelconque des revendications 3 à 5, **caractérisée en ce que** le mélange d'écran solaire, d'agent autobronzant et de pigments contient une proportion de pigments de 25 à 40 % en poids par rapport au mélange total de poudre.

7. Poudre-crème ou poudre compacte utilisée en cosmétique selon l'une quelconque des revendications 1 à 6, **caractérisée en ce que** la teneur en eau de la poudre n'est pas supérieure à 15 % en poids.

8. Poudre-crème ou poudre compacte utilisée en cosmétique selon l'une quelconque des revendications 1 à 7, **caractérisée en ce que** la teneur en agents autobronzants se situe au total dans la plage de 1 à 15 % en poids par rapport à la composition totale.

9. Poudre-crème ou poudre compacte utilisée en cosmétique selon la revendication 7, **caractérisée en ce que** la teneur maximale en DHA se situe à 5% en poids, celle de l'extrait de mahakanni à 10 % en poids et celle de l'extrait de tulipe à 10 % en poids dans le mélange total.

10. Procédé de fabrication de la Poudre-crème ou poudre compacte selon la revendication 1, **caractérisé par**
le mélange de la base de poudre, éventuellement d'autres adjuvants et substances actives utilisées en cosmétique et d'une première part de 90 à 50 % en poids des pigments colorants, avec la cire ou le mélange de cire fondue, puis par l'ajout de l'agent autobronzant choisi parmi des mélanges de dihydroxyacétone (DHA) avec de l'extrait de mahakanni (Eclipta alba Hassk.) et des mélanges de DHA avec de l'extrait de tulipe, et d'une deuxième part de 10 à 50 % en poids des pigments en suspension aqua-alcoolique avec un pH dans la plage 3-4, dans le mélange de cire fondue à une température de 65 à 70°C, par l'agitation de l'ensemble du mélange jusqu'à l'homogénéité et le refroidissement du mélange en maintenant l'homogénéité.
